# FASCICULE DE BREVET EUROPEEN

(11) **EP 2 121 736 B1**
(45) Date de publication et mention de la délivrance du brevet: **30.03.2011**
(21) Numéro de dépôt: 08701640.8
(22) Date de dépôt: 23.01.2008
(51) Int. Cl.: C07K 14/415, C12N 9/10, C07K 14/445, A61K 38/16, C12N 15/62, C07K 19/00, A61P 37/04

(54) **NOUVELLES COMPOSITIONS VACCINALES ANTI PALUDIQUE ET SES UTILISATIONS**
NEUE ANTIMALARIA-IMPFSTOFFZUSAMMENSETZUNGEN UND ANWENDUNGEN DAVON
NEW ANTI-MALARIA VACCINE COMPOSITIONS AND USES THEREOF

(30) Priorité: 23.01.2007 FR 0752837
(43) Date de publication de la demande: 25.11.2009
(73) Titulaire: CENTRE NATIONAL DE LA RECHERCHE SCIENTIFIQUE (CNRS), 75016 Paris (FR); UNIVERSITE DES SCIENCES ET TECHNOLOGIES DE LILLE, 59650 Villeneuve d'Ascq (FR)
(72) Inventeur: TOMAVO, Stanislas, F-59520 Marquette lez Lille (FR); BALL, Steven Graham, F-59830 Bourghelles (FR); D'HULST, Christophe, F-59150 Wattrelos (FR); DAUVILLEE, David, F-59650 Villeneuve d'Ascq (FR)
(74) Mandataire: Warcoin, Jacques
(86) Numéro de dépôt international: PCT/EP2008/050758
(87) Numéro de publication internationale: WO 2008/090174

(56) Documents cités:
- WO-A-00/71734
- WO-A-2004/096861
- Q JI ET AL.: "Microbial starch-binding domains as a tool for targeting proteins to granules during starch biosynthesis" PLANT MOLECULAR BIOLOGY., vol. 51, 2003, pages 789-801, XP002450219 NL SPRINGER, DORDRECHT.

## Description

La présente invention concerne le paludisme et, plus spécifiquement, un polypeptide destiné à la vaccination antipaludique, une composition pharmaceutique le contenant et l'utilisation d'un tel polypeptide pour la préparation d'une composition destinée au traitement prophylactique du paludisme.

Le paludisme, qui résulte d'une infection par un parasite du genre *P*/*asmodium,* demeure à ce jour l'endémie majeure dans le monde avec près de 300 et 500 millions de cas et plus de 2,5 millions de morts par an, dont majoritairement des enfants de moins de cinq ans (World Health Organization Tropical Disease Research, TDR Twelfth Program Report, p. 57-76, 1997). Aujourd'hui, plus de 40% de la population mondiale vit dans les régions où sévit le paludisme.

Différents traitements prophylactiques et thérapeutiques ont été développés contre ce parasite, les molécules les plus efficaces agissant lors de la phase d'infection des globules rouges. On peut citer ainsi la chloroquine (NIVAQUINE), l'halofantrine (HALFAN), la méfloquine (LARIAM) et la quinine ; la quinine-restant à ce jour le médicament de référence. Toutefois, nombre de ces molécules présentent d'importants effets secondaires et on note l'apparition de résistances à certains médicaments antipaludiques développés par le parasite le plus dangeureux *(Plasmodium falciparum)* dans certaines zones géographiques.

Les voies de recherche d'un vaccin antipaludique sont aujourd'hui nombreuses et prés de 40 antigènes utilisables pour l'élaboration d'un vaccin ont pu être identifiés, lesdits antigènes étant fonction du stade de développement du parasite. On peut ainsi distinguer pour le stade intramoustique (stades sexués) : l'antigène Pfg27, Pfs16, Pfs25, Pfs28, Pfs45/48 ou Pfs230 ; pour le stade intravasculaire (sporozoïte) : l'antigène CSP-1, STARP, SALSA ou SSP-2 ; pour le stade intrahépatique : l'antigène LSA-1, EXP-1, LSA-3, STARP, SALSA ou SSP-2 ; et pour le stade intra-érythrocyte (mérozoïte) : l'antigène RAP-1, RAP-2, SERA-1, MSP-1, MSP-2, MSP-3, MSP-4, MSP-5, AMA-1, EMP-1. Pf35, Pf55 ou EBA-175.

À titre d'exemple d'antigène, on peut citer l'antigène membranaire apical 1 (AMA1 ; numéro d'accession CAD42016 à CAD41967, CAD35671 à CAD35504, CAD34791 à CAD34741 et CAD 31724 à CAD31720) qui correspond à une protéine transmembranaire identifiée à l'origine chez *Plasmodium knowlesi* (DEANS et al., Clin. Exp. Immunol., vol.49, p :297-309, 1982 ; DEANS et al., Mol. Biochem. Parasitol., vol.11, p :189-204;1984) et, ultérieurement, dans d'autres espèces appartenant au genre Plasmodium (WATERS et al., J. Biol. Chem., vol.265, p : 17974-17979, 1990). Cette protéine est présente dans les organelles apicaux du mérozoïte du parasite de la malaria au stade schizonte tardif (HEALER et al., Infect. Immun., vol.70, p :5751-5758, 2002 ; BANNISTER et al., J. Cell. Sci., vol.116, p :3825-3834, 2003) ou à la surface du mérozoïte au stade rupture du schizonte et invasion des érythrocytes (NARUM et THOMAS, Mol. Biochem. Parasitol., vol.67, p :59-68, 1994). Il a pu être démontré que la présence d'anticorps dirigés contre l'antigène AMA1 permet d'empêcher l'invasion d'érythrocytes (THOMAS et al., Mol. Biochem. Parasitol., vol.13, p :187-199,1984 ; KOCKEN et al., J. Biol. Chem., vol.273, p :15119-15124,1998 ; HODDER et al., Infect. Immun., vol.69(5), p :3286-94, 2001), suggérant un rôle critique associé à cet antigène de surface. De surcroît, il a été démontré que l'expression de la protéine AMA1 est vitale pour la survie du parasite (TRIGLIA et al., Mol. Microbiol., vol.38, p :706-718, 2000). Finalement, la production d'AMA1 dans différents systèmes a été décrite, mais se révèle problématique du fait de quantités insuffisantes (baculovirus) ou de problèmes de repliement (*E.*coli) lié au grand nombre de ponts disulfures.

On peut également citer l'antigène MSP1 (Merozoite Surface Protein 1 ; numéro d'accession BAA2624 à BAA2604, AAQ20930 à AAQ20923, et AAC69750 à AAC69718) qui est synthétisé au stade schizonte et qui est impliqué notamment dans l'invasion érythrocytaire par le parasite. Au cours de cette dernière, MSP1 subit plusieurs coupures protéolytiques au cours du processus de maturation du mérozoïte. Parmi ces produits de clivage, un fragment correspondant à l'extrémité C-terminale de MSP1 et présentant une masse moléculaire d'environ 42 kDa (MSP1-42 ; HOLDER et al., Parasitology, vol.94, p:199-208, 1987; LYON et al., Proc. Natl. Acad. Sci. USA, vol.83, p:2989-2993, 1986). MSP1-42 reste accroché à la membrane du mérozoïte dans la phase précédent l'invasion. Au moment même de l'invasion, MSP1-42 est lui-même scindé en deux fragments : un fragment N-terminal d'environ 33 kDa et un fragment C-terminal d'environ 19 kDa (MSP1-19 ; BLACKMAN et al., Mol, Biochem. Parasitol., vol.50, p:307-316, 1992). Cette dernière coupure est essentielle pour le succès de l'invasion, bien que le mécanisme du processus n'ait pas encore été élucidé. En raison des difficultés à produire la protéine MSP1 du fait de sa taille importante (environ 200 kDa), les chercheurs ont étudié essentiellement la portion C-terminale qui présente certainement la fonction (inconnue à ce jour) la plus importante. Finalement, la production de protéines recombinantes comprenant la partie C-terminale de MSP1 a été décrite avec notamment une p19 et une p40 fusionnées chacune à une glutathione-S-transférase produite chez *E. coli* (BURGHAUS et al., Infection and Immunity, vol.64, p:3614-3619, 1996 ; KUMAR et al., Molecular Medicine, vol.1(3), p:325-332, 1995), ou une p19 fusionnée avec un polypeptide dérivé d'une anatoxine tétanique et portant des épitopes de cellules T auxiliaires produit chez *S. cerevisiae* (KUMAR *et al.,* 1995, cité précédemment). Toutefois, ces différentes protéines recombinantes ont montré une efficacité variable en terme de production d'anticorps après injection chez le singe.

Finalement, et malgré les efforts importants entrepris par la communauté scientifique internationale, les différents candidats vaccinaux testés n'ont montré qu'une efficacité relative et très limitée pour la protection contre le paludisme. De surcroît, le fait que le paludisme se localise essentiellement au niveau de pays en voie de développement imposent, pour un éventuel vaccin, de répondre à 3 critères essentiels de (1) efficacité immédiate, (2) administration facile et (3) coût faible.

C'est dans ce contexte que les inventeurs ont développé une stratégie originale consistant en l'utilisation de l'amidon comme vecteur portant des épitopes de *Plasmodium falciparum* afin d'élaborer un vaccin.

L'amidon, polymère de réserve par excellence du règne végétal, représente l'une des sources les plus importantes de polysaccharides présents sur terre et peut, notamment, être trouvé dans les plantes (maïs, pomme de terre, blé, riz, orge...), les algues, les microalgues, etc. L'amidon se présente sous forme de grains insolubles, dont la taille peut varier de 0,1 à plusieurs dizaines de µm de diamètre, et se compose de deux sous-fractions polysaccharidiques dénommées amylose et amylopectine représentant environ 25 et 75% en poids du grain d'amidon respectivement. Composées uniquement de résidus de glucose liés par des liaisons alpha-1,4 et branchés en alpha-1,6, ces deux fractions diffèrent aussi bien d'un point de vue architectural que dans la nature des fonctions enzymatiques intervenant dans leur synthèse. Alors que l'amylopectine (la fraction majeure du grain d'amidon qui lui confère son caractère cristallin) nécessite un ensemble complexe d'enzymes pour son élaboration, la formation de l'amylose ne fait intervenir qu'une amidon synthétase particulière appelée GBSS (Granule Bound Starch Synthase).

Plusieurs isoformes de GBSS ont été isolées chez le maïs, le pois, la pomme de terre ou encore le blé (MACDONALD et PREISS, Plant Physiology, vol.78, p: 849-852,1985 ; SMITH, Planta, vol.182, p: 599-604, 1990 ; DRY et al., The Plant Journal, vol.2(2), p: 193-202, 1992 ; DENYER et al., Planta, vol.196, p: 256-265, 1995). Dans tous les cas, c'est la GBSSI qui représente l'isoforme majeure; ladite isoforme étant impliquée dans la formation d'amylose lors la biogenèse du grain d'amidon (TSAI, Biochemical Genetics, vol.11 (2), p:83-95, 1974 ; HOVENKAMP-HERMELINK et al., Theorical and Applied Genetics, vol.75, p:217-221, 1987 ; DELRUE et al., Journal of Bacteriology, vol.174(11), p: 3612-3620, 1992 ; *DENYER et al.*, 1995, précité) et les mutants déficients pour cette enzyme ne contenant que de l'amylopectine.

L'enzyme GBSSI utilise de l'ADP-glucose afin de relier entre eux les résidus de glucose par des liaisons alpha-1,4 donnant naissance à de longues chaînes peu ramifiées caractéristiques de la fraction amylosique. Une particularité remarquable de cette enzyme repose sur la nécessité d'être liée à une matrice polysaccharidique pour effectuer la réaction enzymatique. Ainsi l'enzyme n'est observée que piégée au sein du grain d'amidon et elle représente à elle seule près de 1 % du poids sec de l'amidon dans des conditions de culture optimales à sa production. Cette abondance et la localisation atypique de l'enzyme engendrent de nombreux avantages technologiques. La GBSSI peut ainsi être très aisément purifiée à l'aide de protocoles utilisés en routine aussi bien à l'échelle du laboratoire qu'à celle de l'industrie. L'amidon purifié contenant la protéine peut également être conservé sans dégradation notable pendant des mois à température ambiante. La localisation particulière de la protéine d'intérêt (au sein même du grain d'amidon) permet également de s'affranchir de la présence d'allergènes végétaux souvent observés lors de la préparation de protéines recombinantes chez la plante.

Un ADNc correspondant à la protéine Waxy (par abus de langage, on utilise le terme protéine Waxy pour désigner la GBSSI chez les plantes, la distinguant ainsi des autres GBSS) a été isolé chez le blé,l'orge, le maïs, le riz, la pomme de terre et le pois. Cette protéine d'environ 60 kDa possède une séquence hautement conservée dans le règne végétal (Ainsworth et al., Plant Mol Biol., vol.22(1), p:67-82, 1993). Une exception remarquable est cependant à noter dans le cas de l'algue verte unicellulaire *Chlamydomonas reinhardtii*, où l'enzyme présente une extension carboxy-terminale de 12 kDa de séquence non pertinente provenant certainement d'une fusion avec un autre gène (WATEFELD et al., Eur. J. Biochem., vol.269(15), p : 3810-20, 2002). Une souche mutante de Chlamydomonas isolée par l'équipe de Steven Ball produit une protéine tronquée de 4 kDa de cette extension mais possède toujours sa capacité à se fixer à l'intérieur du grain d'amidon.

Les inventeurs ont fusionné un peptide portant des épitopes de *Plasmodium falciparum* avec la protéine GBSSI de l'algue *Chlamydomonas.* Ils ont alors démontré que la protéine de fusion comprenant le peptide parasitaire MSP1 produite dans l'amidon de l'algue est très immunogénique chez les lapins et les souris conduisant à la production d'anticorps polyclonaux qui sont capables de reconnaître très spécifiquement l'antigène MSP1 natif ainsi que les parasites. Finalement, ces anticorps polyclonaux sont également capables d'inhiber complètement *in vitro,* la pénétration de *Plasmodium falciparum* à l'intérieur des hématies. Ces résultats ouvrent maintenant la voie vers l'expression d'autres antigènes de *Plasmodium* dans l'algue *Chlamydomonas,* en particulier des épitopes théoriquement invariants appartenant aux antigènes de structures apicales, ce qui devrait permettre la réalisation des tests de protection contre l'infection et la maladie chez des modèles murins.

Dans la demande internationale PCT WO 00/71734, les inventeurs ont décrit le principe de protéines de fusion entre une protéine GBSSI tronquée ou non et un polypeptide d'intérêt. Pour autant, ce document ne fait aucun cas de l'utilisation de telle protéines de fusion à des fins vaccinales, pas plus que de la possibilité d'obtenir des protéines de fusion dans lesquelles le polypeptide correspondant à l'épitope présente un repliement correct. Dans tous les cas, ce document ne décrit ni ne suggère des protéines de fusion comprenant des épitopes d'un parasite du genre *Plasmodium* permettant d'obtenir une réponse immune ciblée après injection.

En conséquence, un premier objet de l'invention concerne un polypeptide dont la séquence N-terminale comprend la séquence polypeptidique de la GBSSI (Granule Bound Starch Synthase I), une séquence polypeptidique présentant un pourcentage d'identité d'au moins 80% avec ladite séquence polypeptidique de la GBSSI ou un fragment d'au moins 50 acides aminés de ladite séquence polypeptidique de la GBSSI;
(i) laquelle séquence polypeptidique présente la capacité de se fixer à l'amidon ; et
(ii) la séquence C-terminale comprend une séquence polypeptidique codant pour au moins un épitope d'un antigène d'un parasite du genre *Plasmodium.*

La séquence de la GBSSI dans différentes espèces de plantes, d'algues ou de micro-algues fait partie des connaissances générales de l'homme du métier ou pourra être déterminée par de simples expériences de routine du fait de la conservation de sa séquence.

Par séquence polypeptidique de la GBSSI, on entend la séquence polypeptidique de la GBSSI mature, après clivage du peptide signal du précurseur de la GBSSI.

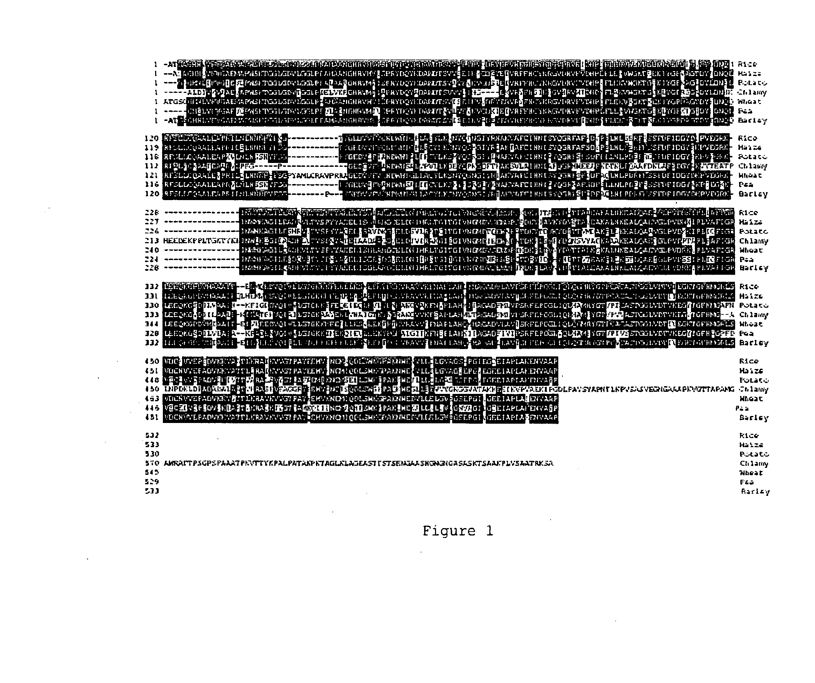

La conservation de séquences de la protéine GBSSI entre les espèces apparaît clairement au regard de la figure 1 qui montre un alignement des séquences de GBSSI matures du blé (*Triticum aestivum* ; numéro d'accession : P27736), de *Chlamydomonas reihnarditii* (numéro d'accession : AAL28128), du maïs (*Zea mays* ; numéro d'accession : P04713), du pois (*Pisum sativum* ; numéro d'accession : Q43092), du riz (*Oriza sativa* ; numéro d'accession : P19395), de l'orge (*Hordeum vulgare* ; numéro d'accession : P09842) et de la pomme de terre (Solanum tuberosum ; numéro d'accession : Q00775).

Avantageusement, la séquence polypeptidique de la GBSSI est choisie dans le groupe comprenant la GBSSI de *Chlamydomonas reinhardtii* (SEQ ID NO :1), de blé (*Triticum aestivum* ; SEQ ID NO :2) , de maïs *(Zea mays* ; SEQ ID NO :3), de pois (*Pisum sativum* ; SEQ ID NO :4), de riz *(Oriza sativa* ; SEQ ID NO :5), d'orge (*Hordeum vulgare* ; SEQ ID NO :6), de pomme de terre (*Solanum* tuberosum ; SEQ ID NO :7), du soja (*Glycine max*; SEQ ID NO :8) et de haricot (*Phaseolus vulgaris* ; SEQ ID NO:9).

De préférence, la séquence polypeptidique de la GBSSI correspond à la séquence polypeptidique de la GBSSI de *Chlamydomonas neinhardtii* (SEQ ID NO :1) et de manière particulièrement préférée à la séquence polypeptidique allant des positions 1 à 527 de la GBSSI de *Chlamydomonas reinhardtii* (SEQ ID NO : 1)

Par fragment de GBSSI, on entend un polypeptide d'au moins 50 acides aminés, à titre d'exemple d'au moins 100 ou 150 acides aminés, de préférence d'au moins 200 acides aminés, à titre d'exemple d'au moins 250 ou 350 acides aminés, et de manière particulièrement préférée un polypeptide d'au moins 400 acides aminés.

À titre d'exemple, les inventeurs ont pu montrer que le fragment allant de la position 1 à la position 438 de la GBSSI mature chez *Chlamydomonas reinharditii* (SEQ ID NO :1). L'homme du métier est capable de déduire au regard de ses seules connaissances générales les fragments correspondants de GBSSI dans d'autres espèces.

De préférence, des fragments de GBSSI capables de se lier à l'amidon sont choisis dans le groupe comprenant la séquence polypeptidique allant des positions 1 à 438 de la GBSSI de *Chlamydomonas reinharditii* (SEQ ID NO :1), 1 à 449 de la GBSSI de blé (*Triticum aestivum;* SEQ ID NO :2), 1 à 437 de la GBSSI de maïs *(Zea mays* ; SEQ ID NO :3), 1 à 432 de la GBSSI de pois (*Pisum sativum ;* SEQ ID NO :4), 1 à 436 de la GBSSI de riz (*Oriza sativa* ; SEQ ID NO :5), 1 à 437 de la GBSSI d'orge (*Hordeum vulgare ;* SEQ ID NO :6), 1 à 434 de la GBSSI de pomme de terre (*Solanum* tuberosum ; SEQ ID NO :7), 1 à 435 de la GBSSI de soja (*Glycine max* ; SEQ ID NO :8) et 1 à 431 de la GBSSI de haricot (*Phaseolus vulgaris* ; SEQ ID NO :9).

Par antigène, on entend par la présente un polypeptide capable de déclencher une réaction immunitaire à médiation humorale ou à médiation cellulaire.

L'homme du métier pourra identifier simplement et au regard de ses connaissances générales un antigène d'un parasite du genre *Plasmodium*, notamment de *Plasmodium falciparum, Plasmodium vivax, Plasmodium ovale* ou *Plasmodium malariae*, de préférence un antigène de *Plasmodium falciparum* ou de *Plasmodium vivax*, et de manière particulièrement préférée un antigène de *Plasmodium falciparum*.

À titre d'exemple de tels antigènes, on peut citer les antigènes suivants : Pfg27/25 (SEQ ID NO :10), Pfs16 (SEQ ID NO:11), Pfs25 (SEQ ID NO :12), Pfs28 (SEQ ID NO :13), Pfs48/45 (SEQ ID NO :14), Pfs230 (SEQ ID NO :15), CSP-1 (SEQ ID NO :16), STARP (SEQ ID NO :17), SALSA (SEQ ID NO :18), SSP-2 (SEQ ID NO : 19), LSA-1 (SEQ ID NO :20), EXP-1 (SEQ ID NO :21), LSA-3 (SEQ ID NO :22), RAP-1 (SEQ ID NO :23), RAP-2 (SEQ ID NO :24), SERA-1 (SEQ ID NO :25), MSP-1 (SEQ ID NO :26), MSP-2 (SEQ ID NO :27), MSP-3 (SEQ ID NO :28), MSP-4 (SEQ ID NO :29), MSP-5 (SEQ ID NO :30), AMA-1 (SEQ ID NO :31), EMP-1 (SEQ ID NO :32) et EBA-175 (SEQ ID NO :33).

Par épitope, on entend une structure présente à la surface de la molécule d'antigène et capable de se fixer à une seule molécule d'anticorps.

Avantageusement, on entend par épitope une séquence polypeptidique dérivée de la séquence polypeptidique d'un antigène d'un parasite du genre *Plasmodium.*

Avantageusement encore, on entend par épitope une séquence polypeptidique d'au moins 6 acides aminés, de préférence d'au moins 8 acides aminés, par exemple d'au moins 10 acides aminés, et de manière particulièrement préférée d'au moins 12 acides aminés.

L'homme du métier, au regard de ses connaissances générales et de simples expériences de routine, sera capable d'identifier dans la séquence d'un antigène d'un parasite du genre *Plasmodium,* la séquence polypeptidique codant pour un épitope reconnu spécifiquement par un anticorps. À titre d'exemple, l'homme du métier pourra utiliser notamment le procédé décrit dans la demande internationale PCT WO02/30964.

Par séquence dérivée, on entend une séquence polypeptidique présentant un pourcentage d'identité d'au moins 70 %, de préférence d'au moins 80% et de manière particulièrement préférée d'au moins 90% avec la séquence polypeptidique d'un antigène d'un parasite du genre *Plasmodium.*

La séquence polypeptidique de l'épitope pourra ainsi comprendre des substitutions (1,2 ou 3) au regard de la séquence polypeptidique d'un antigène d'un parasite du genre *Plasmodium,* de sorte d'améliorer l'ancrage et ainsi la présentation du polypeptide correspondant audit épitope par les molécules du CMH de classe II.

Selon un mode de réalisation préférée, la séquence polypeptidique dudit épitope présente une identité de 100% avec la séquence polypeptidique d'un antigène d'un parasite du genre *Plasmodium.*

Selon un mode de réalisation préféré, ledit antigène correspond à l'antigène MSP1 (SEQ ID NO :26).

De préférence, la séquence polypeptidique codant pour au moins un épitope d'un antigène d'un parasite du genre *Plasmodium* est la séquence SEQ ID NO :34.

Avantageusement, le polypeptide selon l'invention présente la séquence SEQ ID NO :35.

Selon un second mode de réalisation préféré, ledit antigène correspond à l'antigène AMA-1 (SEQ ID NO :31).

De préférence, la séquence polypeptidique codant pour au moins un épitope d'un antigène d'un parasite du genre *Plasmodium* est la séquence SEQ ID NO :36.

Avantageusement, le polypeptide selon l'invention présente la séquence SEQ ID NO :37.

Avantageusement, le polypeptide selon l'invention comprend une séquence polypeptidique de liaison entre les séquences N- et C-terminales.

Un deuxième objet de l'invention porte sur un polynucléotide codant pour un polypeptide tel que décrit précédemment.

Ledit polynucléotide correspond à une séquence d'ADN, de préférence ledit polynucléotide est une séquence d'ADN.

Avantageusement, ledit polynucléotide code pour un polypeptide présentant en outre un peptide signal à son extrémité N-terminale, lequel peptide signal permet le transport intra-cellulaire dudit polypeptide vers les sites de biosynthèse des grains d'amidon. De préférence, ledit peptide signal correspond au peptide signal de GBSSI de l'organisme dans lequel est produit le polypeptide selon l'invention.

À titre d'exemple, on pourra utiliser le peptide signal de GBSSI de *Chlamydomonas reihnarditii* (SEQ ID NO :38), lorsqu'il est envisagé de produire le polypeptide selon l'invention dans cet organisme.

Avantageusement encore, le polynucléotide selon l'invention est lié de façon opérationnelle à une séquence d'expression génique dirigeant l'expression dudit polynucléotide dans une cellule eucaryote, de préférence dans une cellule de plante, d'algue ou de micro-algue. Ladite séquence d'expression génique correspond à toute séquence de régulation, telle qu'une séquence promotrice ou une combinaison entre une séquence promotrice et une séquence activatrice facilitant la transcription et la traduction efficace du polypeptide tel que décrit précédemment. Ladite séquence d'expression génique peut correspondre à une séquence promotrice virale ou eucaryote, constitutive ou inductible. A titre de séquences promotrices utilisables, on peut citer à titre d'exemples, des séquences promotrices eucaryotes de type végétaux supérieurs, tels que les promoteurs 35S et CaMV, ou des séquences promotrices eucaryotes de microalgues, tels que les promoteurs HSP70, Rubisco, ARG7 (arginosuccinate lyase) et NIT1 (nitrate réductase).

Un troisième objet de l'invention concerne un vecteur comprenant le polynucléotide tel que décrit précédemment.

À titre d'exemple de tels vecteurs, on peut citer les plasmides, les phages et les plasmides.

Un quatrième objet de l'invention concerne une cellule transformée par un vecteur tel que décrit précédemment.

De préférence, ladite cellule transformée est une cellule végétale contenant un ou plusieurs polynucléotides tels que décrits précédemment, intégrés dans leur génome ou maintenus de manière stable dans leur cytoplasme, lesdites cellules végétales étant choisies parmi les cellules de plantes, d'algues ou de micro-algues, capables de fabriquer de l'amidon.

La transformation de cellules végétales peut être réalisée selon des techniques bien connues de l'homme du métier. À titre d'exemple, on peut citer la technique d'électroporation, de microinjection cytoplasmique ou nucléaire, de canon à gène ou de transformation à l'aide de bactéries transformée telle que *Agrobacterium tumefaciens.*

Un cinquième objet de l'invention concerne également un organisme transformé tel qu'une plante, une algue ou une microalgue et comprenant une cellule transformée telle que décrite précédemment.

Parmi les plantes, algues ou micro-algues transformées dans le cadre de la présente invention, on peut citer principalement le blé, le maïs, la pomme de terre, le riz, l'orge, l'amarante, les algues du genre Chlamydomonas, notamment *Chlamlydomonas reinhardtii*, les algues du genre Chlorella, notamment *Chlorella vulgaris.*

Un sixième objet de l'invention concerne une composition pharmaceutique comprenant un polypeptide tel que décrit précédemment, éventuellement associé à un support pharmaceutiquement acceptable.

À titre d'exemple de support pharmaceutiquement acceptable, la composition peut comprendre des émulsions, des microémulsions, des émulsions huile dans l'eau, des lipides anhydres et des émulsions eau dans l'huile, ou d'autres types d'émulsions.

La composition selon l'invention peut comprendre en outre un ou plusieurs additifs tels que les diluants, les excipients, les stabilisateurs et les conservateurs. De tels additifs sont bien connus de l'homme du métier et sont décrits notamment dans « Ullmann's Encyclopedia of Industrial Chemistry, 6th Ed.» (différents éditeurs, 1989-1998, Marcel Dekker); et dans "Pharmaceutical Dosage Forms and Drug Delivery System "s (ANSEL et al., 1994, WILLIAMS & WILKINS).

Le polypeptide selon l'invention peut être solubilisé dans un tampon, dans de l'eau ou incorporé dans des émulsions ou des microémulsions. À titre d'exemple de tampons utilisables, on peut citer le tampon phosphate salin (PBS), le sérum artificiel (150 mM NaCl dans de l'eau) et les tampons Tris.

Il existe de nombreuses causes d'instabilité ou de dégradation des polypeptides telles que l'hydrolyse et la dénaturation, pouvant entraîner une diminution de l'induction de la réponse humorale ou cellulaire. Des stabilisateurs peuvent être ajoutés pour diminuer ou prévenir de tels problèmes.

À titre d'exemple de stabilisateurs, on peut citer les monosaccharides, les disaccharides, les polysaccharides, les détergents ioniques et non-ioniques, les sels de métaux alcalins, les phospholipides, les acides gras, les polyols et les peptides stabilisants comme le sérum albumine bovine.

Avantageusement, ledit au moins un polypeptide est associé à des grains d'amidon.

Les inventeurs ont en effet montré que la production du polypeptide selon l'invention en association avec les grains d'amidon permettait d'augmenter sa stabilité.

La composition selon l'invention pourra se trouver alors sous une forme administrable par voie parentérale, notamment par voie intraveineuse, intra-péritonéale ou sous une forme administrable par voie orale.

De préférence, la composition pharmaceutique susmentionnée administrable par voie parentérale, est caractérisée en ce que le diamètre des grains d'amidon est compris entre 0,1 µm et plusieurs µm, notamment entre environ 0,1µm et 10µm. La proportion en poids dudit au moins un polypeptide dans ces grains d'amidon est alors comprise entre 0,1 % et 1 % en poids.

Des grains d'amidon tels que décrits ci-dessus dont les faibles diamètres sont compris entre 0,1 µm et 10 µm, et dans lesquels la proportion en poids dudit au moins un polypeptide tel que décrit précédemment est comprise entre environ 0,1 % et 1 %, sont avantageusement obtenus :
- à partir d'algues ou micro-algues transformées dans le cadre de la présente invention, on peut citer principalement *Chlamydomonas reinhardtii*
- à partir de plantes ou cellules de plantes transformées dans le cadre de la présente invention et choisies pour leur propriété à produire naturellement les grains d'amidon susmentionnés, lesdites plantes étant choisies notamment parmi le riz et l'amarante ;
- à partir de parties de plantes transformées dans le cadre de la présente invention, lesdites parties de ces plantes produisant naturellement les grains d'amidon susmentionnés, telles que les feuilles des plantes ;
- à partir de plantes ou cellules de plantes transformées dans le cadre de la présente invention, ces plantes étant choisies parmi des plantes comportant des mutations telles qu'elles produisent des grains d'amidon de faibles diamètres susmentionnés, notamment à partir des plantes mutantes décrites dans BULEON et al. (Int. J. Biol. Macromolecules, vol.23, p :85-112, 1998) ;
- à partir de plantes ou cellules de plantes transformées dans le cadre de la présente invention, ces plantes étant choisies parmi des plantes transformées à l'aide de séquences nucléotidiques antisens de tout ou partie du gène codant pour l'ADP-glucose pyrophosphorylase nécessaire à la synthèse d'ADP-glucose dans les cellules végétales, notamment à partir des plantes transformées décrites dans MÜLLER-RÖBER et al. (EMBO J., vol.11(4), p :1229-1238,1992).

Avantageusement, dans le cas d'une composition pharmaceutique administrable par voie parentérale, les grains d'amidon sont de préférence choisis parmi ceux de structure amorphe dans le cas où l'on souhaite obtenir une libération rapide du polypeptide tel que décrit précédemment qu'ils contiennent dans le sang du sujet, ou, au contraire parmi ceux de structure cristalline lorsque l'on souhaite libérer progressivement ledit polypeptide dans le sang.

À titre d'illustration, des grains d'amidon amorphes peuvent être obtenus à partir de semences transformées selon l'invention en phase de germination, ou à partir de plantes mutantes particulières telles que décrites par SHANNON et GARWOOD (In starch : Chemistry and Technology, 2nd edition, Academic Press, San Diego, California, p :16-86, 1984), notamment à partir des cultivars mutants tels que"amylose extender"du maïs ou encore tous les cultivars mutants de plantes, algues ou micro-algues dont l'amidon est enrichi en amylose.

À titre d'illustration encore, des grains d'amidon de structure cristalline présentent avantageusement 30 à 35% de cristaux et peuvent être obtenus à partir de semences de plantes, notamment de céréales, venant d'être récoltées et à maturité, ou à partir de plantes mutantes telles que décrites par SHANNON et GARWOOD (précité, 1984), notamment à partir des cultivars mutants tels que "waxy"du maïs ou encore tous les cultivars mutants de plantes, algues ou micro-algues dont l'amidon est dépourvu d'amylose.

De tels grains d'amidon pourront être purifiés à partir des plantes, parties de plantes, algues ou micro-algues à l'aide de techniques bien connues de l'homme du métier. À titre d'exemple de telles techniques, on pourra citer les techniques décrites dans HARRIS (The Chlamydomonas Sourcebook. A Comprehensive Guide to Biology and Laboratory Use. Academic Press, San Diego,CA., 1989) et dans KINDLE (High-frequency nuclear transformation of Chlamydomonas reinhardtii. Genetics, 87, 1228-1232, 1990)

Un septième objet de l'invention porte sur une méthode de traitement prophylactique d'une pathologie associée à un parasite du genre *Plasmodium* comprenant l'administration d'une quantité thérapeutiquement efficace d'une composition telle que décrite précédemment à un sujet.

Tel qu'utilisé dans la présente demande, le terme « sujet » correspond à un mammifère tel qu'un rongeur, un félin, un canin, un primate ou un humain, de préférence ledit sujet est un humain.

Par pathologie associée à un parasite du genre *Plasmodium,* on entend notamment le paludisme.

Par « quantité thérapeutiquement efficace », on entend une quantité permettant d'induire la production d'anticorps neutralisants. L'homme du métier sera à même de déterminer ladite quantité thérapeutiquement efficace au regard de ses connaissances générales et/ou à l'aide de simples expériences de routine.

Un huitième objet de l'invention porte sur l'utilisation d'une composition telle que décrite précédemment pour la fabrication d'un médicament destiné au traitement prophylactique d'un sujet d'une pathologie associée à un parasite du genre *Plasmodium*, de préférence du paludisme.

Selon un mode de réalisation préféré, ladite composition est destinée a une administration par voie parentérale.

Selon un autre mode de réalisation préféré, ladite composition est destinée à une administration par voie orale.

Les exemples qui suivent sont fournis à titre d'illustration et ne sauraient limiter la portée de la présente invention.

### EXEMPLES :

### I- Production de protéines de fusion comprenant la GBSSI et des antigènes de parasite du genre Plasmodium

### I-1 Obtention d'épitopes dérivés des antigènes MSP1 et AMA-1 de parasites du genre Plasmodium

Afin de valider ce concept de vaccination antipaludique, deux polypeptides provenant des antigènes majeurs MSP1 et AMA-1 issus de parasites du genre *Plasmodium* ayant prouvés leur efficacité dans des tests de protection contre le parasite ont été sélectionnés (JOHN et al., J. Immunol., vol.173, p :666-72, 2004 ; MALKIN et al., Infect. Immun., vol.73, p : 3677-85, 2005).

Des réactions de PCR à l'aide d'amorces spécifiques ont permis d'amplifier les polynucléotides codant pour
- le peptide C-terminal de 19 kDa (dépourvu de la séquence glycolipidique GPI) de l'antigène majeur de surface MSP1 (merozoite surface protein 1) de *Plasmodium falciparum* (modèle d'infection humaine), et
- la région centrale de l'antigène AMA-1 (apical major antigen 1) de *Plasmodium berghei* (modèle d'infection murine).

Plus spécifiquement, les amorces 3' utilisées présentaient un site de restriction XhoI et permettaient le clonage ultérieur en phase du polynucléotide obtenu avec la séquence polynucléotidique codant pour la GBSSI. Les amorces 5' présentaient un site de restriction BamHI.

Les fragments PCR MSP1 et AMA-1 de 312 et 336 pb respectivement (SEQ ID NO: 39 et SEQ ID NO:40) ont été purifié puis cloné dans le vecteur pCR2.1 à l'aide du kit TOPO TA CLONING ® (INVITROGEN) selon les instructions du fabricant pour obtenir les plasmides MSP1-pCR2.1 et AMA-1-pCR2.1 respectivment.

### I-2 Extraction du gène de résistance paro^{R} du plasmide pSG2 de Chlamydomonas

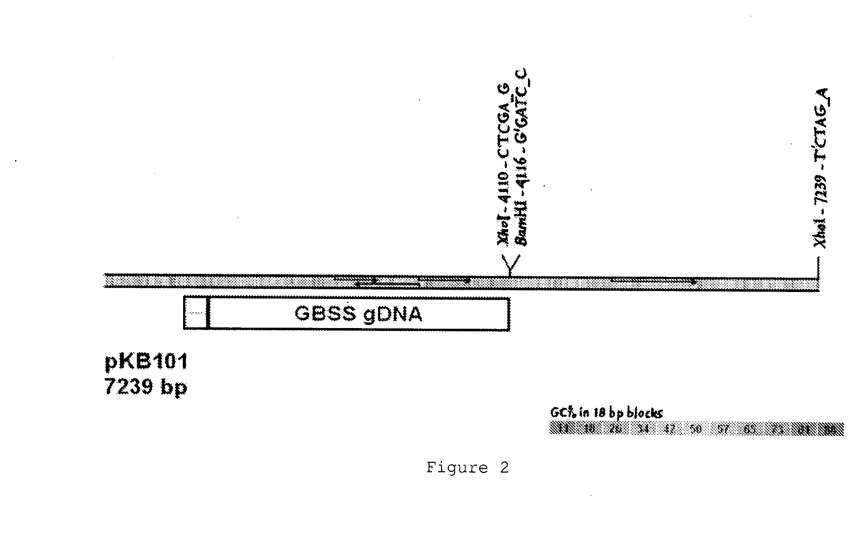

5 µg de plasmide pSG2 a été digéré pendant 2 heures à 37°C avec l'enzyme de restriction XbaI. La bande d'ADN à 2300 pb qui correspond au fragment conférant la - résistance à la paromomycine (paro^{R}) a été excisée et purifiée. Le fragment plasmidique restant pSG2 (200 ng) a été re-circularisé par la T4 DNA ligase (BIOLABS) à 4°C, une nuit, puis le 1/5^{ème} du produit a servi à transformer des bactéries TOP10F' compétentes (INVITROGEN) selon les instructions du fabricant. Afin de vérifier que les bactéries comprennent effectivement le plasmide pKB101 (voir figure 2, laquelle indique l'emplacement de la GBSSI troquée et des sites de clonage ; SEQ ID NO :41) et que le fragment paro^{R} a bien été enlevé, 4 clones ont été choisis et remis en culture dans 5 mL de LB supplémenté en ampicilline (50µg/mL). L'ADN plasmidique a été extrait à l'aide du kit NUCLEOSPIN PLASMID (MACHEREY-NAGEL) selon les instructions du fabricant, puis digéré par l'enzyme XbaI, 2 heures à 37°C afin de vérifier l'absence du fragment paro^{R} de 2300 pb dans le plasmide.

### 1-3 Extraction des inserts MSP1 et AMA-1 des plasmides MSP1 -pCR2.1 et AMA-1-pCR2.1

Les bactéries contenant le plasmide MSP1-pCR2.1 ont été remises en culture dans 50 mL de LB contenant 50µg/mL d'ampicilline. Le plasmide a ensuite été extrait avec le kit HISPEED PLASMID MIDI® (QIAGEN) selon les instructions du fabricant. 5µg de plasmides MSP1-pCR2.1 a été digéré 1 heure à 37 °C par l'enzyme BamHI (BIOLABS), puis 1 heure à 37°C par XhoI (BIOLABS) selon les instructions du fabricant. Le produit de digestion a ensuite été déposé sur gel d'agarose et la bande d'ADN de 300pb correspondant au fragment MSP1 a été purifiée, puis ligaturée avec l'enzyme T4 DNA ligase, comme précédemment, dans le plasmide pSG2 préalablement digéré par Xho/BamHI pour obtenir le plasmide MSP1-pSG2'. Un cinquième du produit de ligature a alors servi à transformer des bactéries TOP10F' compétentes (INVITROGEN) comme précédemment.

Dans plusieurs clones positifs issus de cette transformation, la présence du plasmide MSP1-pSG2' a été confirmé par PCR et séquençage direct de l'ADN.

Le même protocole a été utilisé sur le plasmide AMA-1-pCR2.1 pour obtenir le plasmide AMA1-pSG2', lequel comprend un fragment de 312 pb correspondant à 104 acides aminés de AMA1

### 1-4 Insertion du gène de résistance à la paromomycine (paro^{R}) dans les plasmides MSP1-pSG2' et AMA1-pSG2'

Le plasmide MSP1-pSG2' (5µg) a été digéré par l'enzyme de restriction XbaI pendant 2 heures à 37°C. La linéarisation complète du plasmide a été vérifiée en déposant un aliquote de la digestion sur gel d'agarose 1 %.

Le plasmide MSP1-pSG2' linéarisé a ensuite été traité à la phosphatase alcaline (ROCHE) et le fragment paro^{R} purifié précédemment (cf. I-2) a été inséré dans le plasmide MSP1-pSG2' pour obtenir le plasmide MSP1-pSG2 à l'aide de la T4 DNA ligase (BIOLABS) selon les instructions du fabricant. Un cinquième du produit de ligature a alors servi à transformer des bactéries TOP10F' compétentes (INVITROGEN) et quelques clones positifs ont été digérés par l'enzyme XbaI afin de vérifier la présence d'un insert de 2300 pb.

La même procédure a été utilisée pour la construction du plasmide AMA1-pSG2 à partir du plasmide AMA1-pSG2'.

### 1-5 Transformation de l'algue Chlamydomonas avec les plasmides MSP 1-pSG2 et AMA 1-pSG2

Une souche mutante de Chlamydomonas, dépourvue de GBSS endogène, a été transformée à l'aide de la technique des billes de verre avec 1 µg de plasmide MSP1-pSG2 selon le protocole décrit dans KINDLE (1990, précité).

Brièvement, 300µL d'une suspension cellulaire provenant d'une culture en TAP en phase exponentielle de croissance concentrée 100 fois est vortexée violement en présence d'un microgramme d'ADN pendant 15 secondes. Six cents microlitres de TAP sont ajoutés et la suspension cellulaire est étalée sur boîte (TAP supplémentée de paromomycine à 10µg/mL) pour sélectionner les clones ayant intégré le plasmide MSP1-pSG2. Les clones résistants à la paromomycine apparaissent en une dizaine de jours à 23 degrés et sous lumière continue et sont ensuite criblés afin d'identifier les clones exprimant la protéine de fusion GBSSI-MSP1 (SEQ ID NO: 35).

Le même protocole a été utilisé avec le plasmide AMA1-pSG2 pour obtenir des clones exprimant la protéine de fusion GBSSI-AMA-1 (SEQ ID NO: 37).

### II Expression des protéines GBSSI-MSP1 et GBSSI-AMA-1

L'amidon de différents clones résistants et susceptibles de comprendre la protéine de fusion GBSSI-MSP1 a été purifié comme décrit dans HARRIS (1989, précité).

Brièvement, une culture de l'algue verte *Chlamydomonas reinhardtii* en milieu non carencé (TAP) est centrifugée à 3000 g pendant 10 minutes lorsque la densité cellulaire atteint 3 à 4 millions de cellules par mL. Le culot cellulaire est broyé à l'aide d'une presse de French (10000 PSI). Le lysat est par la suite centrifugé (13000 rpm, 15 minutes) et le culot obtenu contenant l'amidon et les débris cellulaires est conservé. Ce culot est resuspendu dans du percoll (GE HEALTHCARE) 90% puis soumis à une centrifugation (13000 rpm, 45 minutes). Le culot d'amidon obtenu est lavé par de l'eau milliQ deux fois (13000 rpm, 10 minutes).

Le dosage de l'amidon s'effectue à l'aide du kit commercialisé par DIFFCHAMP (ENZYPLUS STARCH®) puis aliquoté par 10mg. L'amidon peut ainsi être conservé à 4 degrés plusieurs mois sans dégradation apparente. Les protéines sont ensuite extraites d'environ 1 mg d'amidon pour chaque clone avec 60 µl de tampon d'extraction (β-mercaptoéthanol 5%(v/v), SDS 2% (p/v)) à 100°C pendant 5 minutes. Après centrifugation à 13000g pendant 10 minutes, le surnageant est récupéré et l'opération est renouvelée une fois avec le culot. Les deux - sumageants sont réunis et sont chargés sur deux gels d'électrophorèse SDS-PAGE. A titre de contrôle, les mêmes opérations sont effectuées sur l'amidon d'une souche de *Chlamydomonas reinhardtii* exprimant la GBSSI sauvage. L'un des deux gels est coloré au bleu de Coomassie et l'autre est transféré sur membrane de nitrocellulose (SCHLEICHER & SCHUELL) selon les instructions du fabricant pour analyse en Western blot avec un anticorps polyclonal dirigé contre le peptide parasitaire de 19 KDa de MSP1 puis avec un anticorps polyclonal spécifique de la GBSSI de *Chlamydomonas reinhardtii.*

Le gel coloré au coomassie a révélé l'existence d'une bande majoritaire à environ 70 kDa pour les algues mutantes transformées et à environ 70 kDa pour l'algue sauvage ce qui correspond à la taille attendue pour les protéines GBSSI-MSP1 et GBSSI respectivement. En effet, le peptide C-terminal de la GBSS a été supprimé et est remplacé par le peptide MSP1, ce qui permet d'avoir une protéine recombinante de taille similaire. La différence entre les transformants exprimant la GBSS1-MSP1 et les souches résistantes ne l'exprimant pas peut être observée par la présence d'une protéine de 70 kDa extraite des grains d'amidon. En outre, les résultats ont montré que lesdites protéines sont bien reconnues par l'anticorps polyclonal dirigé contre la GBSSI. En revanche, seule la protéine majoritaire exprimée dans les souches mutantes et transformées par le vecteur MSP1-pSG2 est reconnue par l'anticorps polyclonal dirigé contre le peptide parasitaire de 19 KDa de MSP1.

Les résultats ont également montré que la protéine de fusion GBSS-AMA1 était aussi exprimée avec succès dans l'amidon d'algues transformées par le vecteur AMA1-pSG2.

### III Production d'anticorps polyclonaux dirigés spécifiquement contre la protéine de fusion GBSS-MSP1-19:

Une dizaine de milligrammes d'amidon, contenant la protéine de fusion GBSS-MSP1 et purifié comme décrit précédemment, a été utilisé pour immuniser cinq souris Balb/c avec de l'adjuvant complet de Freund suivi de deux rappels avec de l'adjuvant incomplet à un mois d'intervalle chacun. Deux semaines après la dernière immunisation, les sérums des cinq souris ont été collectés.

En parallèle, et à titre de contrôle négatif, une dizaine de milligrammes d'amidon contenant la GBSS sauvage et purifié comme précédemment, a été utilisé pour immuniser cinq souris Balb/c selon le protocole décrit précédemment.

L'existence d'anticorps dirigés contre la protéine de fusion GBSS-MSP1 dans le sérum collecté a été confirmé par une analyse par Western blot dont les résultats ont montré que les sérums obtenus chez les souris immunisées avec la GBSS-MSP1 reconnaissent spécifiquement et uniquement l'antigène majeur de surface MSP1 de 190 kDa ainsi que ses différents peptides protéolytiques, tels que les peptides de 70 kDa et de 42 kDa, ainsi que le fragment membranaire des mérozoïtes infectants, le peptide de 19 kDa. Comme attendu, les sérums produits contre la GBSS seule ne reconnaissent eux aucun antigène parasitaire, ce qui confirme la spécificité de la reconnaissance des antigènes parasitaires contenant les épitopes du peptide 19 kDa.

La spécificité du sérum polyclonal anti-GBSS-MSP1 à l'encontre du peptide de 19 kDa de MSP1 a également été confirmé par la technique d'immunofluorescence indirecte (IFA). En effet, et comme attendu, les résultats ont montré que ledit sérum reconnaissait spécifiquement la membrane et la surface de *Plasmodium falciparum* à l'intérieur des hématies infectées alors que les sérums anti-GBSS seuls sont totalement négatifs.

Finalement, l'expression d'une protéine de fusion dans l'amidon permet donc non seulement sa purification simple mais également de tester aisément son immunogénicité puisque par une simple absorption, l'amidon sera transformé dans l'organisme en glucose et les protéines antigéniques libérées lors de ce processus deviendront donc accessibles au système immunitaire.

### IV Inhibition de l'invasion des hématies par Plasmodium falciparum:

Une culture de *Plasmodium falciparum* synchrone contenant des schizontes a été utilisée. 900 microL d'hématies à 50% d'hématocrite et 1% de parasitémie contenant uniquement des schizontes a été resuspendu dans 20 mL de RPMI complet. Ensuite, 200 microL de la suspension ont été distribués dans chaque puit d'une plaque de culture à 96 puits et 10, 15 ou 20 microL de sérum sain ou anti GBSS-MSP1 ont été rajoutés dans des séries de puits contenant la suspension parasitaire. Pour chaque concentration, 4 puits ont été testés.

Les résultats ont montré que les anticorps polyclonaux spécifiques de la GBSS-MSP1 ont complètement inhibé (100% d'inhibition) l'invasion des globules rouges par *Plasmodium falciparum.* En revanche, les anticorps polyclonaux dirigés contre l'amidon seul n'ont révélé aucune inhibition de l'invasion des globules rouges par *Plasmodium falciparum.*

Finalement, ces résultats démontrent que l'amidon contenant la GBSS-MSP1 peut donc éliciter une réponse humorale contenant des anticorps polyclonaux qui neutralisent la pénétration du *Plasmodium falciparum* à l'intérieur des globules rouges.

## Revendications

1. Un polypeptide dont :
la séquence N-terminale comprend la séquence polypeptidique de la GBSSI (Granule Bound Starch Synthase I), une séquence polypeptidique présentant un pourcentage d'identité d'au moins 80% avec ladite séquence polypeptidique de la GBSSI ou un fragment d'au moins 50 acides aminés de ladite séquence polypeptidique de la GBSSI;
(i) laquelle séquence polypeptidique présente la capacité de se fixer à l'amidon ; et
(ii) la séquence C-terminale comprend une séquence polypeptidique codant pour au moins un épitope d'un antigène d'un parasite du genre *Plasmodium* choisi de préférence dans le groupe comprenant *Plasmodium falciparum*, *Plasmodium vivax*, *Plasmodium ovale* et *Plasmodium malariae.*

2. Le polypeptide selon la revendication 1, **caractérisé en ce que** la séquence polypeptidique de la GBSSI est choisie dans le groupe comprenant la GBSSI de *Chlamydomonas reinhardtii* (SEQ ID NO :1), de blé (*Triticum aestivum* ; SEQ ID NO :2), de maïs (*Zea mays ;* SEQ ID NO :3), de pois (*Pisum sativum ;* SEQ ID NO :4), de riz (*Oriza sativa ;* SEQ ID NO :5), d'orge (*Hordeum vulgare* ; SEQ ID NO :6), de pomme de terre (Solanum tuberosum ; SEQ ID NO :7), du soja (*Glycine max;* SEQ ID NO :8) et de haricot *(Phaseolus vulgaris ;* SEQ ID NO :9), de préférence la séquence polypeptidique de la GBSSI correspond à la séquence polypeptidique de la GBSSI de *Chlamydomonas reinhardtii* (SEQ ID NO :1).

3. Le polypeptide selon l'une quelconque des revendications précédentes, **caractérisé en ce que** les fragments de GBSSI capables de se lier à l'amidon sont choisis dans le groupe comprenant la séquence polypeptidique allant des positions 1 à 438 de la GBSSI de *Chlamydomonas reinharditii* (SEQ ID NO :1), 1 à 449 de la GBSSI de blé (*Triticum aestivum* ; SEQ ID NO :2), 1 à 437 de la GBSSI de maïs (*Zea mays ;* SEQ ID NO :3), 1 à 432 de la GBSSI de pois *(Pisum sativum ;* SEQ ID NO :4), 1 à 436 de la GBSSI de riz (*Oriza sativa ;* SEQ ID NO :5), 1 à 437 de la GBSSI d'orge (*Hordeum vulgare* ; SEQ ID NO :6), 1 à 434 de la GBSSI de pomme de terre (Solanum tuberosum ; SEQ ID NO :7), 1 à 435 de la GBSSI de soja (*Glycine max*; SEQ ID NO :8) et 1 à 431 de la GBSSI de haricot (*Phaseolus vulgaris ;* SEQ ID NO :9).

4. Le polypeptide selon l'une quelconque des revendications précédentes **caractérisé en ce que** ledit antigène est choisi dans le groupe comprenant les antigènes Pfg27/25 (SEQ ID NO :10), Pfs16 (SEQ ID NO :11), Pfs25 (SEQ ID NO :12), Pfs28 (SEQ ID NO :13), Pfs48/45 (SEQ ID NO :14), Pfs230 (SEQ ID NO :15), CSP-1 (SEQ ID NO :16), STARP (SEQ ID NO :17), SALSA (SEQ ID NO :18), SSP-2 (SEQ ID NO :19), LSA-1 (SEQ ID NO :20), EXP-1 (SEQ ID NO :21), LSA-3 (SEQ ID NO :22), RAP-1 (SEQ ID NO :23), RAP-2 (SEQ ID NO :24), SERA-1 (SEQ ID NO :25), MSP-1 (SEQ ID NO :26), MSP-2 (SEQ ID NO :27), MSP-3 (SEQ ID NO :28), MSP-4 (SEQ ID NO :29), MSP-5 (SEQ ID NO :30), AMA-1 (SEQ ID NO :31), EMP-1 (SEQ ID NO :32) et EBA-175 (SEQ ID NO :33), de préférence ledit antigène correspond à l'antigène MSP1 (SEQ ID NO :26) ou à l'antigène AMA-1 (SEQ ID NO :31).

5. Le polypeptide selon la revendication 4, **caractérisé en ce que** ledit épitope présente une séquence polypeptidique d'au moins 6 acides aminés, de préférence d'au moins 8 acides aminés, dérivée de la séquence polypeptidique dudit antigène et de préférence, ledit épitope consiste en une séquence polypeptidique présentant un pourcentage d'identité d'au moins 70 %, de préférence d'au moins 80%, avec la séquence polypeptidique dudit antigène.

6. Le polypeptide selon la revendication 5, **caractérisé en ce que** la séquence polypeptidique de l'épitope comprend des substitutions au regard de la séquence polypeptidique dudit antigène, de sorte d'améliorer l'ancrage et ainsi la présentation du polypeptide correspondant audit épitope par les molécules du CMH de classe II.

7. Le polypeptide selon la revendication 5, **caractérisé en ce que** ledit antigène correspond à l'antigène MSP1 (SEQ ID NO :26) et **en ce que** la séquence polypeptidique codant pour au moins un épitope dudit épitope est la séquence SEQ ID NO :34, de préférence ledit polypeptide présente la séquence SEQ ID NO :35.

8. Le polypeptide selon la revendication 5, **caractérisé en ce que** ledit antigène correspond à l'antigène AMA-1 (SEQ ID NO :31) et **en ce que** la séquence polypeptidique codant pour au moins un épitope dudit antigène est la séquence SEQ ID NO :36, de préférence ledit polypeptide présente la séquence SEQ ID NO :37.

9. Le polypeptide selon l'une quelconque des revendications précédentes, **caractérisé en ce qu'**il comprend une séquence polypeptidique de liaison entre les séquences N- et C-terminales, de préférence ladite séquence de liaison comprend un site de clivage peptidique permettant de libérer l'épitope après purification dudit polypeptide selon l'invention.

10. Un polynucléotide codant pour un polypeptide selon l'une quelconque des revendications 1 à 9.

11. Un vecteur comprenant un polynucléotide selon la revendication 10.

12. Une cellule transformée par un vecteur selon la revendication 11.

13. Un organisme, à l'exception de l'Homme, transformé, **caractérisé en ce qu'**il comprend une cellule selon la revendication 12.

14. Une composition pharmaceutique comprenant au moins un polypeptide selon l'une quelconque des revendications 1 à 9, éventuellement associé à un support pharmaceutiquement acceptable, ledit polypeptide étant de préférence associé à des grains d'amidon.

15. Utilisation d'un polypeptide selon l'une quelconque des revendications 1 à 9 pour la fabrication d'un médicament destiné au traitement prophylactique d'un sujet d'une pathologie associée à un parasite du genre *Plasmodium,* de préférence du paludisme.

## Claims

1. A polypeptide wherein:
the N-terminal sequence comprises the polypeptide sequence of GBSSI (Granule Bound Starch Synthase I), a polypeptide sequence presenting a percentage of identity of at least 80% with said polypeptide sequence of GBSSI or a fragment of at least 50 amino acids of said polypeptide sequence of GBSSI;
(i) said polypeptide sequence being capable of binding to starch; and,
(ii) the C-terminal sequence comprises a polypeptide sequence coding for at least one epitope of an antigen of a parasite of the genus *Plasmodium* preferably chosen from the group comprising *Plasmodium falciparum, Plasmodium vivax, Plasmodium ovale* and *Plasmodium malariae.*

2. The polypeptide according to claim 1, **characterised in that** the GBSSI polypeptide sequence is chosen from the group comprising GBSSI of *Chlamydomonas reinhardtii* (SEQ ID NO: 1), wheat (*Triticum aestivum;* SEQ ID NO: 2), maize (Zea *mays;* SEQ ID NO: 3), pea (*Pisum sativum;* SEQ ID NO: 4), rice (*Oriza sativa;* SEQ ID NO: 5), barley (*Hordeum vulgare;* SEQ ID NO: 6), potato (*Solanum tuberosum;* SEQ ID NO: 7), soya (*Glycine* max; SEQ ID NO: 8) and bean (*Phaseolus vulgaris*; SEQ ID NO: 9), preferably the GBSSI polypeptide sequence corresponds to the GBSSI polypeptide sequence of *Chlamydomonas reinhardtii* (SEQ ID NO: 1).

3. The polypeptide according to any of the preceding claims, **characterised in that** the GBSSI fragments able to bind to starch are chosen from the group comprising the polypeptide sequence from positions 1 to 438 of *Chlamydomonas reinhardtii* GBSSI (SEQ ID NO: 1), 1 to 449 of wheat GBSSI (*Triticum aestivum;* SEQ ID NO: 2), 1 to 437 of maize GBSSI (Zea *mays;* SEQ ID NO: 3), 1 to 432 of pea GBSSI (Pisum *sativum;* SEQ ID NO: 4), 1 to 436 of rice GBSSI (Oriza sativa; SEQ ID NO: 5), 1 to 437 of barley GBSSI (*Hordeum vulgare;* SEQ ID NO: 6), 1 to 434 of potato GBSSI (*Solarium tuberosum;* SEQ ID NO: 7), 1 to 435 of soya GBSSI (*Glycine max;* SEQ ID NO: 8) and 1 to 431 of bean GBSSI (*Phaseolus vulgaris;* SEQ ID NO: 9).

4. The polypeptide according to any of the preceding claims, **characterised in that** said antigen is chosen from the group comprising antigens Pfg27/25 (SEQ ID NO: 10), Pfs16 (SEQ ID NO: 11), Pfs25 (SEQ ID NO: 12), Pfs28 (SEQ ID NO: 13), Pfs48/45 (SEQ ID NO: 14), Pfs230 (SEQ ID NO: 15), CSP-1 (SEQ ID NO: 16), STARP (SEQ ID NO: 17), SALSA (SEQ ID NO: 18), SSP-2 (SEQ ID NO: 19), LSA-1 (SEQ ID NO: 20), EXP-1 (SEQ ID NO: 21), LSA-3 (SEQ ID NO: 22), RAP-1 (SEQ ID NO: 23), RAP-2 (SEQ ID NO: 24), SERA-1 (SEQ ID NO: 25), MSP-1 (SEQ ID NO: 26), MSP-2 (SEQ ID NO: 27), MSP-3 (SEQ ID NO: 28), MSP-4 (SEQ ID NO: 29), MSP-5 (SEQ ID NO: 30), AMA-1 (SEQ ID NO: 31), EMP-1 (SEQ ID NO: 32) and EBA-175 (SEQ ID NO: 33), preferably said antigen corresponds to antigen MSP-1 (SEQ ID NO: 26) or to antigen AMA-1 (SEQ ID NO: 31).

5. The polypeptide according to claim 4, **characterised in that** said epitope has a polypeptide sequence of at least 6 amino acids, preferably of at least 8 amino acids, derived from the polypeptide sequence of said antigen and, preferably, said epitope consists of a polypeptide sequence presenting a percentage of identity of at least 70%, preferably of at least 80%, with the polypeptide sequence of said antigen.

6. The polypeptide according to claim 5, **characterised in that** the polypeptide sequence of the epitope comprises substitutions in view of the polypeptide sequence of said antigen, of a sort that improve anchoring and thus the presentation of the polypeptide corresponding to said epitope by class II MHC molecules.

7. The polypeptide according to claim 5, **characterised in that** said antigen corresponds to antigen MSP-1 (SEQ ID NO: 26) and **in that** the polypeptide sequence coding for at least one epitope of said antigen is sequence SEQ ID NO: 34, preferably said polypeptide has sequence SEQ ID NO: 35.

8. The polypeptide according to claim 5, **characterised in that** said antigen corresponds to antigen AMA-1 (SEQ ID NO: 31) and **in that** the polypeptide sequence coding for at least one epitope of said antigen is sequence SEQ ID NO: 36, preferably said polypeptide has sequence SEQ ID NO: 37.

9. The polypeptide according to any of the preceding claims, **characterised in that** it comprises a linker polypeptide sequence between the N- and C-terminus sequences, preferably said linker sequence comprises a peptide cleavage site making it possible to release the epitope after purification of said polypeptide according to the invention.

10. A polynucleotide coding for a polypeptide according to any of claims 1 to 9.

11. A vector including a polynucleotide according to claim 10.

12. A cell transformed by a vector according to claim 11.

13. An organism, excluding human, transformed, **characterised in that** it comprises a cell according to claim 12.

14. A pharmaceutical composition comprising at least one polypeptide according to any of claims 1 to 9, optionally combined with a pharmaceutically acceptable medium, said polypeptide being preferably combined with starch grains.

15. Polypeptide according to any of claims 1 to 9 for the manufacture of a drug for the prophylactic treatment of a pathology associated with a parasite of the genus *Plasmodium,* preferably malaria.

## Patentansprüche

1. Polypeptid, dessen N-terminale Sequenz die Polypeptidsequenz der GBSSI (stärkekorngebundene Stärkesynthase 1), eine Polypeptidsequenz, die mindestens 80% Identität zu der Polypeptidsequenz der GBSSI aufweist, oder ein Fragment von mindestens 50 Aminosäuren der Polypeptidsequenz der GBSSI umfasst;
(i) die Polypeptidsequenz die Fähigkeit aufweist, an die Stärke zu binden; und
(ii) die C-terminale Sequenz eine Polypeptidsequenz umfasst, die für mindestens ein Epitop eines Antigens eines Parasiten der Gattung *Plasmodium* codiert, der bevorzugt ausgewählt ist aus der Gruppe umfassend *Plasmodium falciparum, Plasmodium vivax, Plasmodium ovale* und *Plasmodium malariae.*

2. Polypeptid nach Anspruch 1, **dadurch gekennzeichnet, dass** die Polypeptidsequenz der GBSSI ausgewählt ist aus der Gruppe bestehend aus der GBSSI von *Chlamydomonas reinhardtii* (SEQ ID NO:1), Weizen (*Triticum aestivum;* SEQ ID NO:2), Mais (Zea mays; SEQ ID NO:3), Erbse (*Pisum sativum;* SEQ ID NO:4), Reis (*Oriza sativa;* SEQ ID NO:5), Gerste (*Hordeum vulgare;* SEQ ID NO:6), Kartoffel (*Solanum tuberosum;* SEQ ID NO:7), Soja (*Glycine max;* SEQ ID NO:8) und Bohne (*Phaseolus vulgaris;* SEQ ID NO:9), wobei die Polypeptidsequenz der GBBSI bevorzugt der Polypeptidsequenz der GBBSI von *Chlamydomonas reinhardtii* (SEQ ID NO:1) entspricht.

3. Polypeptid nach einem der vorangehenden Ansprüche, **dadurch gekennzeichnet, dass** die zur Bindung an Stärke fähigen Fragmente der GBSSI ausgewählt sind aus der Gruppe bestehend aus der Polypeptidsequenz, die sich erstreckt von den Positionen 1 bis 438 der GBSSI von *Chlamydomonas reinhardtii* (SEQ ID NO:1), 1 bis 449 der GBSSI von Weizen (*Triticum aestivum;* SEQ ID NO:2), 1 bis 437 der GBSSI von Mais (Zea mays; SEQ ID NO:3), 1 bis 432 der GBSSI der Erbse (*Pisum sativum;* SEQ ID NO:4), 1 bis 436 der GBSSI von Reis (*Oriza sativa;* SEQ ID NO:5), 1 bis 437 der GBSSI von Gerste (*Hordeum vulgare;* SEQ ID NO:6), 1 bis 434 der GBSSI der Kartoffel (*Solanum tuberosum;* SEQ ID NO:7), 1 bis 435 der GBSSI von Soja (*Glycine max;* SEQ ID NO:8) und 1 bis 431 der GBSSI der Bohne (*Phaseolus vulgaris;* SEQ ID NO:9).

4. Polypeptid nach einem der vorangehenden Ansprüche, **dadurch gekennzeichnet, dass** das Antigen ausgewählt ist aus der Gruppe bestehend aus den Antigenen Pfg27/25 (SEQ ID NO:10), Pfs16 (SEQ ID NO:11), Pfs25 (SEQ ID NO:12), Pfs28 (SEQ ID NO:13), Pfs48/45 (SEQ ID NO:14), Pfs230 (SEQ ID NO:15), CSP-1 (SEQ ID NO:16), STARP (SEQ ID NO:17), SALSA (SEQ ID NO:18), SSP-2 (SEQ ID NO:19), LSA-1 (SEQ ID NO:20), EXP-1 (SEQ ID NO:21), LSA-3 (SEQ ID NO:22), RAP-1 (SEQ ID NO:23), RAP-2 (SEQ ID NO:24), SERA-1 (SEQ ID NO:25), MSP-1 (SEQ ID NO:26), MSP-2 (SEQ ID NO:27), MSP-3 (SEQ ID NO:28), MSP-4 (SEQ ID NO:29), MSP-5 (SEQ ID NO:30), AMA-1 (SEQ ID NO:31), EMP-1 (SEQ ID NO:32) und EBA-175 (SEQ ID NO:33), wobei das Antigen bevorzugt dem Antigen MSP-1 (SEQ ID NO:26) oder dem Antigen AMA-1 (SEQ ID NO:31) entspricht.

5. Polypeptid nach Anspruch 4, **dadurch gekennzeichnet, dass** das Epitop eine Polypeptidsequenz von mindestens 6 Aminosäuren, bevorzugt von mindestens 8 Aminosäuren aufweist, die von der Polypeptidsequenz des Antigens stammt, und das Epitop aus einer Polypeptidsequenz besteht, die mindestens 70%, bevorzugt mindestens 80% Identität mit der Polypeptidsequenz des Antigens aufweist.

6. Polypeptid nach Anspruch 5, **dadurch gekennzeichnet, dass** die Polypeptidsequenz des Epitops Substitutionen, die die Polypeptidsequenz des Antigens betreffen, umfasst, um die Verankerung und somit die Präsentation des dem Epitop entsprechenden Polypeptids durch die MHC-Klasse II-Moleküle zu verbessern.

7. Polypeptid nach Anspruch 5, **dadurch gekennzeichnet, dass** das Antigen dem Antigen MSP-1 (SEQ ID NO:26) entspricht und dass die Polypeptidsequenz, die für mindestens ein Epitop des Antigens codiert, die Sequenz SEQ ID NO:34 ist, wobei das Polypeptid bevorzugt die Sequenz SEQ ID NO:35 aufweist.

8. Polypeptid nach Anspruch 5, **dadurch gekennzeichnet, dass** das Antigen dem Antigen AMA-1 (SEQ ID NO:31) entspricht und dass die Polypeptidsequenz, die für mindestens ein Epitop des Antigens codiert, die Sequenz SEQ ID NO:36 ist, wobei das Polypeptid bevorzugt die Sequenz SEQ ID NO:37 aufweist.

9. Polypeptid nach einem der vorangehenden Ansprüche, **dadurch gekennzeichnet, dass** es zwischen der N-terminalen und der C-terminalen Sequenzen eine Verbindungspolypeptidsequenz umfasst, wobei die Verbindungssequenz bevorzugt eine Peptidspaltstelle umfasst, die die Freisetzung des Epitops nach der Aufreinigung des erfindungsgemäßen Polypeptids erlaubt.

10. Polynucleotid, das für ein Polypeptid nach einem der Ansprüche 1 bis 9 codiert.

11. Vektor, der ein Polynucleotid nach Anspruch 10 umfasst.

12. Zelle, die mit einem Vektor nach Anspruch 11 transformiert ist.

13. Transformierter Organismus, mit Ausnahme des Menschen, der **dadurch gekennzeichnet ist, dass** er eine Zelle nach Anspruch 12 umfasst.

14. Pharmazeutische Zusammensetzung, das mindestens ein Polypeptid nach einem der Ansprüche 1 bis 9 umfasst, welches gegebenenfalls an einen pharmazeutisch verträglichen Träger gebunden ist, wobei das Polypeptid bevorzugt an Stärkekörner gebunden ist.

15. Polypeptid nach einem der Ansprüche 1 bis 9 zur Herstellung eines Arzneimittels, für die prophylaktische Behandlung einer Erkrankung, die mit einem Parasiten der Gattung *Plasmodium* in Zusammenhang steht, wobei die Erkrankung bevorzugt Malaria ist.
